# EUROPEAN PATENT APPLICATION

(11) **EP 2 213 233 A1**
(43) Date of publication of application: **04.08.2010**
(21) Application number: 09425028.9
(22) Date of filing: 29.01.2009
(51) Int. Cl.: A61B 6/00

(54) **X-ray apparatus comprising an improved control unit**

(71) Applicant: Vivi S.r.L., 20124 Milano (IT)
(72) Inventor: Villa, Alfio, 20144 Milano (IT)
(74) Representative: Lunati & Mazzoni S.r.L.

(57) **Abstract**

There is disclosed an X-ray apparatus (1), comprising: a support device (2), an X-ray device (3), suitable to be positioned in proximity to a patient and to irradiate at least a portion of the patient to take the X-ray image, an articulated arm (4), connected to the support device (2) and to the X-ray device (3), and suitable to allow positioning of the X-ray device (3), a control unit including adjustment means (6), suitable to allow adjustment of the X-ray apparatus (1) by a user and signalling means (8), suitable to signal the status of the X-ray apparatus, the signalling means (8) comprising a plurality of LEDs (9) positioned inside a diffuser layer (10), made of at least partly transparent polymer material and suitable to diffuse the light coming from the LEDs (9) in various directions, the diffuser layer (10) realizing a signalling surface (11) producing at least part of the outer surface of the support device (2).

## Description

The present invention relates to an X-ray apparatus comprising an improved control unit, in particular for a dental X-ray apparatus, of the type specified in the preamble of Claim 1.

X-ray apparatuses, in particular for dental X-rays, are currently known.

These comprise a support device which supports an articulated arm which, in turn, supports an X-ray device suitable to emit X-rays at the teeth or at a portion of a patient to be X-rayed.

Generally, the support device is constrained to a wall or to a stand and comprises a control unit equipped with control and signalling members, suitable to determine operation of the X-ray apparatus.

The operator using an X-ray apparatus therefore adjusts it through specific controls placed on the support device, places the X-ray apparatus in proximity to the mouth, or to the portion of the patient to be X-rayed, checks that the support device signals that the apparatus is functioning correctly and takes the X-ray image.

In fact, it is necessary to check that the X-ray device is ready to emit X-rays, or is preparing to emit them and that no errors, technical problems or the like are signalled.

The aforesaid prior art presents some important drawbacks.

In fact, this continuous checking operation is relatively complex for the user, who must guide and handle the X-ray device and simultaneously must continuously check the support device.

The X-ray device and the support device are in fact separated in space through the articulated arm and must remain separated so that the former can be moved and positioned in proximity to the mouth or portion of the body of a patient to be X-rayed, and the latter remains constrained to a fixed portion and to the power network and the like.

In this situation the technical aim underlying the present invention is to devise an X-ray apparatus capable of substantially overcoming the aforesaid drawbacks.

Within said technical aim an important object of the invention is to produce an X-ray device which allows the user to check the steps of the operation while operating the X-ray device.

Another important object of the invention is to obtain an X-ray apparatus that is simple to use.

Last but not least object of the invention is to produce an inexpensive X-ray apparatus.

The technical aim and the objects specified are achieved by an X-ray apparatus according to the appended Claim 1.

Preferred embodiments are highlighted in the dependent claims.

The characteristics and advantages of the invention are clarified below by the detailed description of a preferred embodiment of the invention, with reference to the accompanying drawings, wherein:

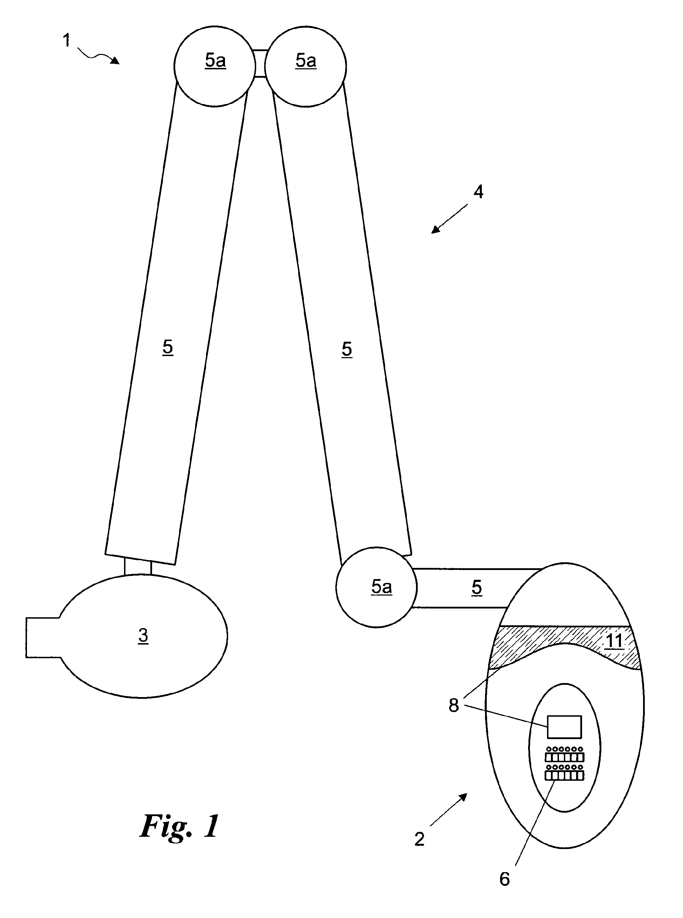

**Fig. 1** shows a dental X-ray apparatus according to the invention;
**Fig. 2** shows a portion of the X-ray apparatus according to the invention; and
**Fig. 3** is the section III-III indicated in Fig. 2.

With reference to the figures cited, the X-ray apparatus according to the invention is indicated as a whole with the reference number **1.**

It comprises, summarily, a support device **2,** an X-ray device **3,** in particular intended for dental X-rays, and an articulated arm **4** connected to the support device 2 and to the X-ray device 3.

The X-ray apparatus 1 is, in particular, a dental X-ray device 2, therefore suitable to take X-rays of the mouth of a patient to examine the teeth or dental prostheses also inside the jaw or under the gums.

In particular, the support device 2 is structured with a compact body and realizes the base of the X-ray apparatus and is constrained to a wall or to a stand.

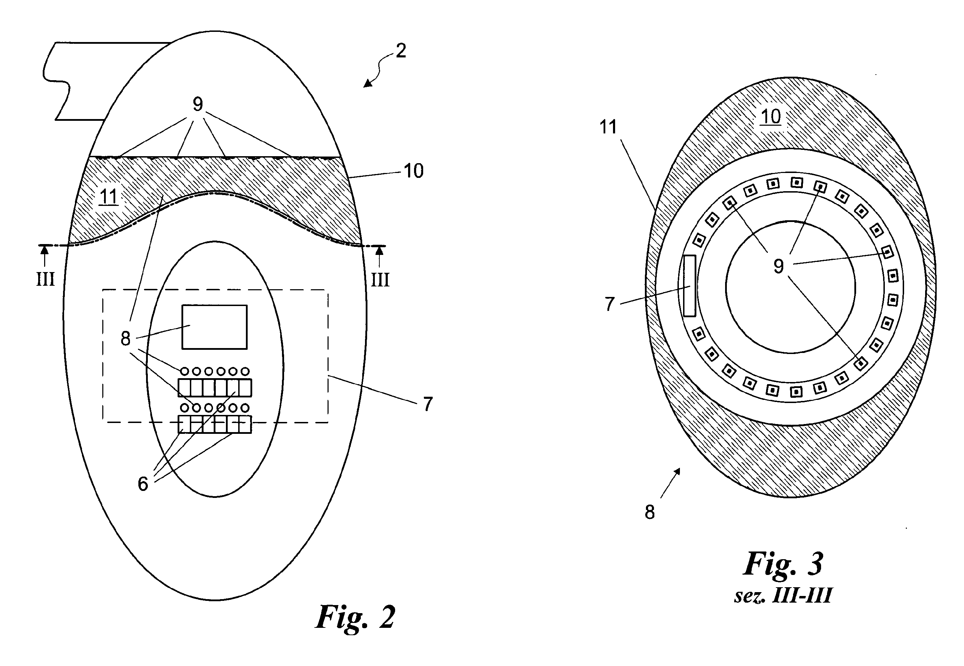

The articulated arm 4 is connected to this support device 2 and preferably comprises two or three segments **5** connected through mechanical joints **5a** such as hinges or the like and therefore allows ample freedom of movement of the X-ray device 3, as shown in Fig. 2.

The X-ray device 3, itself known, must in fact be placed in proximity to the portion of the patient to be X-rayed and therefore in particular in proximity to the tooth or group of teeth to be X-rayed.

This X-ray device 3 is in fact suitable to emit photons or the like, and in particular X-rays, to produce the X-ray image.

The support device 2 comprises a control unit including adjustment means **6** of the apparatus 1.

These adjustment means 6 are realized by push-buttons, knobs or levers or the like, and are suitable to adjust at least part of the settings of the apparatus 1, in particular switching and preparing to take the X-ray image, adjustments relative to the number of X-ray images, the power, the type of tooth and other adjustments still.

The adjustment means 6 are then connected to an electronic processor 7 or the like, present inside the device 1 and connected to the X-ray device through specific cables or connections preferably inside the articulated arm 3.

The control unit also comprises signalling means **8** suitable to signal the status of the X-ray apparatus 1, and in particular switching, the various settings, whether the X-ray device 3 is in the preparation or charging step prior to taking the X-ray, whether there are problems related to the X-ray image and the like.

The signalling means 8 comprise a plurality of LEDs **9,** preferably of different colours, more preferably three colours.

The LEDs 9 are preferably positioned inside the support device 2 and shielded by a diffuser layer **10,** made of at least partly transparent polymer material and suitable to diffuse the light coming from the single LEDs 9 in various directions.

The diffuser layer 10 is preferably at a distance from the LEDs of less than 5 cm and more preferably between 1 cm and 3 cm.

In substance, this diffuser layer 10 is realized by a shaped plate having a rough surface and therefore suitable to diffuse the light coming from the LEDs 9 in the environment and in various directions.

This diffuser layer 10 is also appropriately made of at least partly transparent polymer material, and preferably polycarbonate, and has a thickness in the order of millimetres, and preferably between 0.5 mm and 2 mm.

The diffuser layer 10 realizes a signalling surface **11** which produces at least part of the outer surface of the support device 2.

In particular, the signalling surface 11 extends in the horizontal plane for an angular distance greater than a straight angle, even more preferably greater than three right angles and even better substantially for a round angle.

Operation of an X-ray apparatus 1, described above in structural terms, is as follows.

For a user to take an X-ray image of a patient, the apparatus 1 is switched on and set through said control unit, in particular through the adjustment means 6 positioned on the support device 2.

The user then manoeuvres the X-ray device 3 and positions it in the area of the patient to be X-rayed. This user then waits for the X-ray device 3 to charge, checks that there are no signals or faults and takes the X-ray image.

In particular, by means of the signalling means 8, the user checks the main signals and variables, for example switching of the device, charging in progress or complete charging of the device 3 for emitting X-rays, signalling of malfunctions or faults. These data are highlighted by illuminating a specific colour, and if necessary constant or flashing signals of the signalling surface 11, illuminated by the LEDs 9.

The light signal coming from the signalling surface 11 is transmitted to the user regardless of his/her position in the room. In fact, as previously described, the signalling surface 11 extends for a large angular distance in the horizontal plane.

In particular, any angular areas not covered by illumination of the signalling surface 11 are positioned in the angular portion extending on the wall to which the base 2 is constrained.

Instead, in vertical direction, the user is always positioned more or less at the same height, dictated by the position of the patient on a specific chair or the like.

Moreover, the light signal does not impede or annoy either user or patient, as it is a diffused signal and not pointed and dazzling.

The invention achieves important advantages.

In fact, the X-ray apparatus 1 allows the user to check the support device 2, and the signals coming from it, while operating the X-ray device 3.

Another advantage is realized by the fact that the X-ray apparatus 1 is simple to use. In fact, it does not comprise additional portions and is very intuitive, as for example, a diffused green colour is immediately associated with correct operation, a yellow light with a condition of charging or a fault and a white light with the fact the apparatus is simply switched on.

Moreover, the fact that LEDs 9 of three colours are provided allows numerous signals to be produced, using the three colours, by changing these in various ways and by using constant or intermittent light.

Finally, the X-ray apparatus 1 is inexpensive and simple.

The invention is susceptible to modifications and variants falling within the inventive concept. All the details can be replaced by equivalent elements and the materials, the shapes and dimensions can be any.

## Claims

1. An X-ray apparatus (1), comprising: a support device (2), substantially defining a single body, an X-ray device (3), suitable to be positioned in proximity to a patient and to irradiate at least a portion of said patient to take said X-ray image, and a control unit including adjustment means (6), suitable to allow adjustment of said X-ray apparatus (1) by a user, and signalling means (8), suitable to signal the status of said X-ray apparatus, an articulated arm (4), connected to said support device (2) and to said X-ray device (3), and suitable to allow said positioning of said X-ray device (3), **characterized in that** said signalling means (8) comprise a plurality of LEDs (9) positioned inside a diffuser layer (10), made of at least partly transparent polymer material and suitable to diffuse the light coming from said LEDs (9) in various directions, said diffuser layer (10) realizing a signalling surface (11) producing at least part of the outer surface of said X-ray apparatus (1).

2. Device according to claim 1, wherein said signalling surface (11) defines an outer portion of said control device (2).

3. Device according to one or more of the preceding claims, wherein said control unit is included in said support device (2).

4. Device according to one or more of the preceding claims, wherein said signalling surface (11) extends in the horizontal plane for an angular distance greater than a straight angle.

5. Device according to claim 4, wherein said signalling surface (11) extends in the horizontal plane for an angular distance greater than three right angles.

6. Device according to claim 5, wherein said signalling surface (11) extends in the horizontal plane for an angular distance substantially of one round angle.

7. Device according to one or more of the preceding claims, wherein said LEDs (9) are positioned at distances of less than 5 cm from said diffuser layer (10).

8. Device according to claim 7, wherein said LEDs (9) are positioned at distances between 1 cm and 3 cm from said diffuser layer (10).

9. Device according to one or more of the preceding claims, wherein said diffuser layer (10) is realized by a shaped plate having a rough surface.

10. Device according to one or more of the preceding claims, wherein said diffuser layer (10) is made of polycarbonate.

11. Device according to one or more of the preceding claims, wherein said diffuser layer (10) has a thickness between 0.5 mm and 2 mm.

12. Device according to one or more of the preceding claims, comprising LEDs (9) of three different colours.
